Europäisches Patentamt

European Patent Office

Office européen des brevets

(11) Veröffentlichungsnummer: **0 222 159 B1**

# EUROPÄISCHE PATENTSCHRIFT

(45) Veröffentlichungstag der Patentschrift: **18.09.91**

(51) Int. Cl.⁵: **A61F 2/34**, A61F 2/32

(21) Anmeldenummer: **86113911.1**

(22) Anmeldetag: **07.10.86**

(54) **Hüftpfanne für eine Hüftgelenk-Endoprothese.**

(30) Priorität: **09.10.85 DE 3535959**

(43) Veröffentlichungstag der Anmeldung:
**20.05.87 Patentblatt 87/21**

(45) Bekanntmachung des Hinweises auf die
Patenterteilung:
**18.09.91 Patentblatt 91/38**

(84) Benannte Vertragsstaaten:
**AT BE CH DE ES FR GB IT LI LU NL**

(56) Entgegenhaltungen:
**DE-B- 2 314 175**
**US-A- 4 051 559**

(73) Patentinhaber: **orthoplant Endoprothetik
GmbH
Leerkämpe 12
W-2800 Bremen 66(DE)**

(72) Erfinder: **Schelhas, Klaus-Dieter
Leerkämpe 12
W-2800 Bremen 66(DE)**
Erfinder: **Biehl, Gerd, Prof. Dr. med.
Böcklinstrasse 14
W-5000 Köln 41(DE)**

(74) Vertreter: **Eisenführ, Speiser & Strasse
Martinistrasse 24
W-2800 Bremen 1(DE)**

## Beschreibung

Die Erfindung betrifft eine Hüftgelenk-Endoprothesen-Pfanne, mit einer Außenpfanne mit halbkugelförmiger oder kegelstumpfförmiger Außenform, mit einer in die Außenpfanne einsetzbaren halbkugelförmigen Innenpfanne, und mit einem außen an der Außenschale umlaufenden selbstschneidenden Gewinde zur zementfreien Verankerung der Pfanne in dem Knochengewebe, wobei das Gewinde über die gesamte axiale Gewindelänge hinweg einen annähernd konstanten Außendurchmesser aufweist.

Aus der US-A-4 051 559 ist eine derartige HüftgelenkEndoprothesen-Pfanne bekannt, bei welcher das Gewinde der Außenpfanne eine im wesentlichen konstante Gewindetiefe besitzt. Bei dieser bekannten Ausgestaltung des Gewindes ist die Verankerung der Pfanne im Bereich des Pfannenscheitels, wo sich die Kraftübertragung vom Femurkopf in das Becken konzentriert, oftmals nicht befriedigend, und es kann daher insbesondere bei längerer Benutzung zu unerwünschten Lockerungen in der Pfanne kommen.

Aus der DE-AS 24 11 617 ist eine Hüftgelenk-Endoprothesen-Pfanne bekannt, bei welcher neben dem Innendurchmesser oder Kerndurchmesser des Gewindes auch der Außendurchmesser des Gewindes - an den Außenkanten des Gewindeprofils - zur Stirn- oder Scheitelfläche der Pfanne hin ständig abnimmt. Das Gewindeprofil entspricht dem Gewindeprofil einer Knochenschraube und bleibt über die gesamte axiale Gewindelänge hinweg etwa konstant, um ein leichtes Einsetzen der Pfanne in das entsprechend angefräste Acetabulum ohne Vorschneiden eines Gewindes im Knochengewebe zu ermöglichen; die Gewindetiefe ist daher ebenfalls konstant. Es hat sich gezeigt, daß der Halt derartiger zementfrei eingesetzter Pfannen insbesondere nach längerer Benutzungsdauer nicht immer ganz zufriedenstellend ist. Insbesondere ist die Verankerung im Bereich des Scheitels der Pfanne, wo sich der natürliche oder prothetische Femurkopf abstützt, den erheblichen, über die Pfanne in den Beckenknochen eingeleiteten Kräften oftmals nicht gewachsen, wodurch es über kürzere oder längere Zeit zu den unerwünschten Lockerungserscheinungen kommen kann.

Aus der DE-OS 29 11 754 oder der DE-OS 26 45 101 sind Hüftgelenk-Endoprothesenpfannen bekannt, die vom Pfannenrand zum Scheitel hin verlaufende Ausnehmungen aufweisen, in welche nach dem Einoperieren das Knochengewebe einwachsen soll, um die Haftung der Pfannen im Knochengewebe zu erhöhen.

Aufgabe der Erfindung ist es demgegenüber, eine Hüftgelenk-Endoprothesen-Pfanne der eingangs genannten Art derart weiterzubilden, daß die tragende Fläche der Pfanne in dem umgebenden Knochengewebe vergrößert wird.

Diese Aufgabe wird bei der Hüftgelenk-Endoprothesen-Pfanne der eingangs genannten Art erfindungsgemäß dadurch gelöst, daß das Gewinde eine zum Scheitel der Pfanne hin zunehmende Gewindetiefe besitzt.

Da die Außenkante des Gewindes im wesentlichen auf einem Kreiszylinder liegt, während der Innendurchmesser oder Kerndurchmesser zum Scheitel der Pfanne hin stetig abnimmt, nimmt die tragende Fläche des Gewindeprofils vom kreisförmigen Pfannenrand bis zum Scheitel der Pfanne hin ständig zu. Das im Beckenknochen um die Acetabulumhöhle zur Verfügung stehende Knochengewebe wird dadurch zur Bildung des formschlüssigen Pfannen/Knochengewebe-Verbundes besonders gut ausgenutzt und ein wirksames Ineinandergreifen von Knochensubstanz und Gewinde erzielt. Aufgrund der sich zum Scheitel hin vergrößernden Gewindetiefe kann erforderlichenfalls auch die axiale Gewindelänge verringert, und auch in einem vergleichsweise flachen Becken, bei dem die bekannten Hüftpfannen mit einem Teil ihres Gewindes aus dem Beckenknochen hervorstehen würden, eine gute Verankerung erzielt werden. Bevorzugt besitzt das Gewinde ein unsymmetrisches Profil mit einer in Gewindeachsrichtung zum Scheitel der Pfanne hin versetzten Gewinde-Aussenkante. Besonders bevorzugt sind dabei die das Gewindeprofil begrenzenden Gewindeflanken zur Ebene der ringförmigen Pfannenkante hin konvex gekrümmt.

Die Aussenkante des Gewindeprofils besitzt also bei dieser Ausführungsform einen grösseren Abstand von der Ebene der Pfannenkante als der Profilansatz. Diese Ausführungsform besitzt den Vorteil, dass die Pfanne bei der körpergewichtsbedingten Belastung und der dabei vorhandenen grossen Kraftkomponenten in Gewindeachsrichtung fest im Knochengewebe verankert bleibt und nicht - wie bei den bekannten symmetrischen Gewindeprofilen - das umgebende Knochengewebe radial nach aussen presst und sich dadurch lockert.

Gemäss einer besonders bevorzugten Ausführungsform der Erfindung besitzt die Aussenpfanne aussenliegende Ausnehmungen, welche das Gewinde durchbrechen, die Elastizität der Pfanne erhöhen und andererseits mit Knochengewebe vollwachsen und dadurch das Herausdrehen der Pfanne verhindern. Erfindungsgemäss sind die Ausnehmungen an der Aussenfläche der Aussenpfanne gegen die Ebene der Pfannenkante spitzwinklig angeordnet, sie verlaufen also zum Beispiel auf Grosskreisen, welche mit seitlichem Abstand am Scheitel der Pfanne vorbeilaufen. Dadurch ist die Verankerung des Gewindes über den gesamten Umfang der Pfanne besser verteilt als bei den bekannten Pfannen, welche Ausnehmungen auf durch den Scheitel verlaufenden Grosskreisen besitzen.

Vorteilhafte Weiterbildungen der Erfindung sind durch die Merkmale der Unteransprüche gekennzeichnet.

Im folgenden wird ein Ausführungsbeispiel der Erfindung anhand der Zeichnung näher erläutert.

Es zeigen:

Fig. 1     einen Querschnitt durch die Hüftgelenk-Endoprothesen-Pfanne;

Fig. 2     eine Aussenansicht der Hüftgelenk-Endoprothesen-Pfanne gemäss Fig. 1.

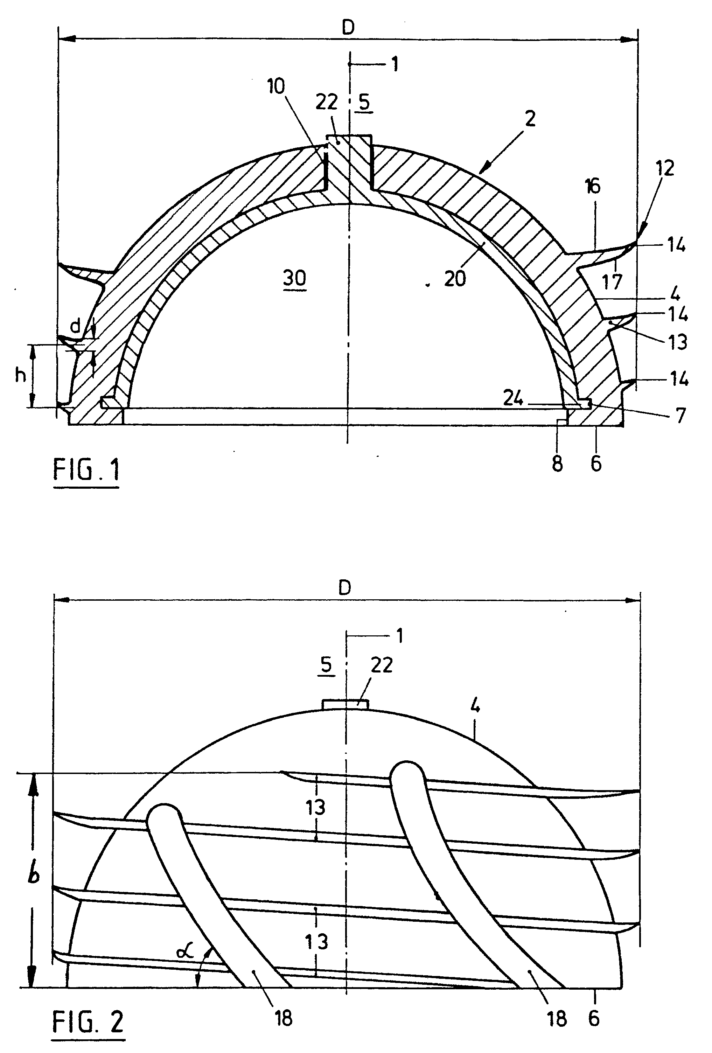

Die Fig. 1 und 2 zeigen eine Hüftgelenk-Endoprothesen-Pfanne 2 im Schnitt und in Frontansicht. Die Pfanne 2 enthält eine Aussenpfanne 4, vorzugsweise aus Metall bzw. aus Keramik, die eine halbkugelförmige Aussenform und eine halbkugelförmige Innenkammer 30 besitzt, in die eine halbkugelförmige Innenpfanne 20 aus Kunststoffmaterial oder dergleichen passend einsetzbar ist. Zur Verankerung der Innenpfanne 20 besitzt die Aussenpfanne 4 im Bereich der ringförmigen Pfannenkante 6 eine Ringnut 7, in welche ein entsprechender Ringansatz 24 der Innenpfanne 20 hineinrastet. Ausserdem besitzt die Aussenpfanne 4 an ihrem Scheitel 5 eine zentrale Scheitelöffnung 10, vorzugsweise von nicht kreisförmigem Querschnitt, in die ein entsprechender Scheitelansatz 22 der Innenpfanne 20 zur Drehsicherung hineinragt. Alternativ kann auch die Aussenpfanne 4 am Scheitel 5 geschlossen sein, und sie kann einen zentralen auswärts ragenden Zentrieransatz aufweisen, um die Pfanne 2 beim Einoperieren in die gewünschte Lage zentrieren zu können.

Der Scheitel 5 der Aussenpfanne kann auch abgeschnitten sein, so dass sich eine relativ grosse kreisförmige Scheitelöffnung ergibt. In dieser Ausführungsform (nicht dargestellt) der Pfanne besitzt die Aussenpfanne 4 eine ringförmige Gestalt, und die Innenpfanne 20 ragt mit einem Kugelabschnitt durch die Scheitelöffnung der Aussenpfanne hindurch. Diese Ausführungsform dient besonders zum Einoperieren in flache Becken, bei denen nur eine geringe Knochentiefe vorhanden ist.

Zur zementfreien Verankerung der Pfanne 2 ist aussen an der Aussenschale 4 ein umlaufendes selbstschneidendes Gewinde 12 angeformt, welches eine relativ grosse Steigung h aufweist, die ein Mehrfaches der Profildicke d am Profilansatz ist. Während die Aussenform der Aussenpfanne 4 als Halbkugel ausgebildet ist - oder in alternativen Ausführungformen (nicht dargestellt) auch die Form eines Kegelstumpfabschnitts aufweisen kann - bleibt der Aussendurchmesser D des Gewindes 12 über die gesamte axiale Gewindelänge b hinweg konstant, so dass die Aussenkante 14 des Gewindes 12 auf einem Kreiszylinder vom Durchmesser D liegt. Das Gewinde 12 besitzt ein unsymmetrisches Profil 13, bei dem die Gewinde-Aussenkante 14 - in Gewindeachsrichtung - gegenüber dem Profilansatz zum Scheitel 5 der Pfanne hin versetzt ist. Die beiden Gewindeflanken 16 und 17, welche das Gewindeprofil 13 begrenzen, besitzen eine zur Ebene der ringförmigen Pfannenkante 6 hin konvexe Krümmung. Die so ausgebildeten Gewindeflanken 16, 17 besitzen zum Scheitel 5 hin eine zunehmend grössere Fläche, und die Aussenkante 14 des Gewindes 12 besitzt von der Ebene der Pfannenkante 6 einen grösseren Abstand als der Ansatz des Gewindes 12 an der Aussenpfanne 4.

Die Aussenpfanne 4 besitzt aussenliegende Ausnehmungen 18, welche gegen die Ebene der Pfannenkante 6 unter einem spitzen Winkel $\alpha$ das Gewinde 12 schneiden. Die Ausnehmungen 18 können in die Pfannenwand ein vorgegebenes Maß hineinragen, alternativ kann ihre Tiefe so bemessen sein, dass sie direkt an der Oberfläche der Pfannenwand oder noch in dem Gewindeprofil 13 enden.

## Patentansprüche

1. Hüftgelenk-Endoprothesen-Pfanne, mit einer Außenpfanne mit halbkugelförmiger oder kegelstumpfförmiger Außenform, mit einer in die Außenpfanne einsetzbaren halbkugelförmigen Innenpfanne, und mit einem außen an der Außenschale umlaufenden selbstschneidenden Gewinde zur zementfreien Verankerung der Pfanne in dem Knochengewebe, wobei das Gewinde über die gesamte axiale Gewindelänge (b) hinweg einen annähernd konstanten Außendurchmesser (D) aufweist, dadurch gekennzeichnet, daß das Gewinde (12) eine zum Scheitel (5) der Pfanne hin zunehmende Gewindetiefe besitzt.

2. Pfanne nach Anspruch 1, dadurch gekennzeichnet, daß das Gewinde (12) ein unsymmetrisches Profil (13) mit einer in Gewindeachsrichtung zum Scheitel (5) der Pfanne hin versetzten Gewinde-Außenkante (14) aufweist.

3. Pfanne nach Anspruch 2, dadurch gekennzeichnet, dass die das Profil (13) des Gewindes (12) begrenzenden Flanken (16, 17) zur Ebene der ringförmigen Pfannenkante (6) hin konvex gekrümmt sind.

4. Pfanne nach einem der vorstehenden Ansprüche, dadurch gekennzeichnet, dass die Steigung (h) des Gewindes (12) ein Vielfaches der am Profilansatz gemessenen Stärke (d) des Gewindeprofils (13) ist.

5. Pfanne nach einem der vorstehenden Ansprüche, dadurch gekennzeichnet, dass die Aussenpfanne (4) aussenliegende Ausnehmungen (18) aufweist, welche gegen die Ebene der Pfannenkante (6) unter einem spitzen Winkel (α) das Gewinde (12) unterbrechen.

## Claims

1. A cup for a hipjoint endoprosthesis, having an outer cup with a hemispherical or frustoconical outer shape and a hemispherical inner cup which may be inserted in the outer cup, and having a selftapping thread running round the outside of the outer cup for anchoring the cup without cement in the bone tissue, the thread exhibiting along its whole axial length (b) an approximately constant outer diameter (D), characterized in that the thread (12) has a depth which increases towards the apex (5) of the cup.

2. A cup as in Claim 1, characterized in that the thread (12) exhibits an asymmetrical profile (13) having an outer edge (14) offset in the direction of the axis of the thread towards the apex (5) of the cup.

3. A cup as in Claim 2, characterized in that the flanks (16, 17) bounding the profile (13) of the thread (12) are curved convexly away from the plane of the annular edge (6) of the cup.

4. A cup as in one of the preceding Claims, characterized in that the pitch (h) of the thread (12) is a multiple of the thickness (d) of the thread profile (13) measured at the root of the thread.

5. A cup as in one of the preceding Claims, characterized in that the outer cup (4) exhibits recesses (18) lying on the outside, which interrupt the thread (12) at an acute angle (α) to the plane of the edge (6) of the cup.

## Revendications

1. Cupule d'endo-prothèse de l'articulation de la hanche, comportant une cupule extérieure présentant une forme extérieure en demi-sphère ou en tronc de cône, une cupule intérieure en forme de demi-sphère pouvant se loger dans la cupule extérieure, ainsi qu'un filetage autotaraudant s'étendent sur la périphérie à l'extérieur de la coque extérieure et destiné à l'ancrage sans ciment de la cupule dans le tissu osseux, le filetage présentant sur toute sa longueur axiale (b), un diametre extérieur (D) sensiblement constant, caractérisée en ce que le filetage (12) présente une profondeur de filet croissante en direction du sommet (5) de la cupule,

2. Cupule selon la revendication 1, caractérisée en ce que le filetage (12) présente un profil (13) non symétrique avec un bord extérieur de filetage (14) déporté vers le sommet (5) de la cupule, dans la direction de l'axe du filetage.

3. Cupule selon la revendication 2, caractérisée en ce que les flancs (16, 17) délimitant le profil (13) du filetage (12), présentent une courbure convexe en direction du plan du bord de cupule (6) de forme annulaire.

4. Cupule selon l'une des revendications précédentes, caractérisée en ce que le pas (h) du filetage (12) est un multiple de l'épaisseur (d) du profil de filetage (13), mesurée à la base de ce profil.

5. Cupule selon l'une des revendications précédentes, caractérisée en ce que la cupule extérieure (4) présente des évidements (18) situés à l'extérieur, qui interrompent le filetage (12) et forment un angle aigu (α) avec le plan du bord de cupule (6).

FIG. 1

FIG. 2